(19) Europäisches Patentamt / European Patent Office / Office européen des brevets

(11) **EP 1 957 087 B1**

(12) **EUROPEAN PATENT SPECIFICATION**

(45) Date of publication and mention
of the grant of the patent:
**02.11.2016 Bulletin 2016/44**

(21) Application number: **06799307.1**

(22) Date of filing: **18.10.2006**

(51) Int Cl.:
*A61K 31/714* (2006.01)     *A61K 45/06* (2006.01)
*A61K 9/127* (2006.01)     *A61P 17/00* (2006.01)

(86) International application number:
**PCT/KR2006/004233**

(87) International publication number:
**WO 2007/066889 (14.06.2007 Gazette 2007/24)**

(54) **COMPOSITIONS FOR EXTERNAL APPLICATION CONTAINING ADENOSYL COBALAMIN FOR USE IN THE TREATMENT OF ATOPIC DERMATITIS**

ADENOSYLCOBALAMIN ENTHALTENDE ZUSAMMENSETZUNG ZUR TOPISCHEN ANWENDUNG ZUR BEHANDLUNG DER ATOPISCHEN DERMATITIS

COMPOSITIONS POUR APPLICATION EXTERNE CONTENANT DE L'ADENOSYL COBALAMINE, DESTINEES AU TRAITEMENT DE LA DERMATITE ATOPIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.12.2005 KR 20050120648**

(43) Date of publication of application:
**20.08.2008 Bulletin 2008/34**

(73) Proprietors:
- **KOREA RESEARCH INSTITUTE OF CHEMICAL TECHNOLOGY**
  **Daejeon 305-343 (KR)**
- **HanAll Biopharma Co., Ltd.**
  **Daejeon 306-120 (KR)**

(72) Inventors:
- **SHIN, Byung Cheol**
  **Daejeon 305-755 (KR)**
- **SEONG, Hasoo**
  **Daejeon 305-743 (KR)**
- **LEE, Aeri**
  **Incheon 407-041 (KR)**
- **KONG, Jae Yang**
  **Daejeon 305-761 (KR)**
- **CHEON, Hyae Gyeong**
  **Daejeon 305-772 (KR)**
- **CHO, Young Sik**
  **Daejeon 305-308 (KR)**
- **JUN, Sung Soo**
  **Gyeonggi-do 463-050 (KR)**
- **JO, Young Gwan**
  **Daejeon 305-811 (KR)**

(74) Representative: **WSL Patentanwälte Partnerschaft mbB**
**Postfach 6145**
**65051 Wiesbaden (DE)**

(56) References cited:
**EP-A2- 0 220 030     EP-A2- 0 256 472**
**WO-A1-99/11242     WO-A1-03/057192**
**WO-A1-2005/094842     WO-A2-03/000010**
**JP-A- 2002 234 845     US-B1- 6 274 564**

Remarks:
The file contains technical information submitted after the application was filed and not included in this specification

## Description

## TECHNICAL FIELD

The present invention relates to a composition for external application for use in the treatment of atopic dermatitis, and particularly to a composition comprising adenosylcobalamin (coenzyme B 12) as an active ingredient, which constitutes a coenzyme of cobalamin in pinocytosis, thereby enabling the improvement of conventional preparation containing cobalamin and its derivative. And this shows delayed pharmaceutical action and is very low in bioavailability as more than 90% of cobalamin or its derivative relative is excreted relative to 100% of administered dosage due to its high molecular weight.

## BACKGROUND ART

Dermatitis is an inflammation caused by various external or internal reasons, and is usually referred to as eczema and includes atopic dermatitis, contagious dermatitis and seborrheic dermatitis.

Although atopic dermatitis is known to be related to immunoglobulin (IgE), it is not certain up to the present of what causes atopic dermatitis. An atopic dermatitis displays symptoms due to external factors, such as various antigens, as it has oversensitive atopic characteristic to specific substance. The region with the symptoms of atopic dermatitis is mainly focused on the face in infancy such as in the form of facial rubefaction, exudative inflammation and desquamation, as well as being very itch. Although the region with the symptoms is usually limited to face in infancy, the symptoms gradually spread to arms and legs, and develop papule pilaris (i.e. atopic skin). Although there are many cases where dermatitis is cured before 12 years old, adults with dermatitis have lichen symptoms on the face, breast and the back of the neck besides arms and legs. This may develop into childhood asthma, and it may take a long period of time until dermatitis may be completely cured. However, there are also many cases where dermatitis returns or relapses and is not completely cured.

An antihistaminic agent and steroid are currently used to treat dermatitis including atopic dermatitis. An antihistaminic agent is usually used to suppress itching, and some of the examples include promethamine hydrochloride, chlorophenylamine maleate, diphenhydramine hydrochloride and mequitazine. Steroid has various side effects despite its remarkable clinical efficacy, and some of the examples include hydrocortisone butyrate, dexametasone valerate, betametasone dipropionate, chlorobetasole propionate and prednisolone. Considering the therapeutical efficacy, the medicine for external application (e.g. ointments) is the most effective and there is no substitute known for this form of medicine. Furthermore, along with the therapeutical effect, the aforementioned medicines are known to have side effects, such as induced infection, secondary adrenocortical insufficiency, diabetes, peptic ulcer, hirsutism, alopecia and pigmentation, etc. In particular, the medicines for external application such as ointments show serious side effects such as skin thinning or shrinking and flushing due to direct influence of the medicine on the skin. Therefore, there is urgent need for stable medicine with less side effects than the conventional dermatitis medicines.

Cobalamin or vitamin B12 is soluble in aqueous solution with the complicate structure, which is one of vitamin B group found in foods. The basic chemical structure of cobalamin comprises two moieties, i.e. porphyrin cyclic structure and nucleotide with alpha-glycoside bonds. The porphyrin cylic structure include four 5,6-dimethylbenzimidazole rings, four nitrogen atoms of which form a coordinate covalent bond with cobalt ion to provide a chelate compound. Cyanocobalamin is a cobalamin where the cobalt atom is bound with cyano group, and the structure without this cyano group is important nutritionologically as well as biochemically. In other words, the cyano group is removed before the activation in a body, and cobalamin changes into co-enzyme and cobalamin enzyme.

Human cannot synthesize a porphyrin cyclic structure, and hence totally depends on foods for the vitamin B12. Although only microorganisms may synthesize a basic cobalamin molecule, cells of all the mammals can change cobalamin into coenzymes, i.e. adenosylcobalamin and methylcobalamin. Hydroxocobalamin, methylcobalamin and adenosylcobalamin are the three types of cobalamin that are separated from the mammal tissues most frequently. However, only methylcobalamin and adenosylcobalamin may act as a supplemental factor in human enzyme. Adenosylcobalamin constitutes components in cells and exists in mitochondria, while methylcobalamin is usally found either in body fluid such as serum and cerebral spinal fluids or in cytoplasm. Cobalamin and its derivatives are reported to have an activity of suppressing dermatitis, especially an inflammation of atopic eczema, which is known to be caused by the production of inflammatory cytokine such as interleukin (IL)-1 alpha, IL-2, IL-6 and interferon (IFN)-gamma [Yamashiki M., Nishimura A., Kosaka Y.; J. Clin. Lab. Immunol.; 1992; 37; 173-182]. Furthermore, cobalamin and its derivatives are the main cause for rubefaction and itching in atopic dermatitis, and are reported to effectively suppress the generation of NO, which is induced by inflammatory cytokine [Stacker M., Pieck C., Stoerb C., Niedner R., Hartung J., Altmeyer P.; Br. J. Dermatol.; 2004; 150; 977-983]. As described above, there have been attempts made to prepare medicine for external application based on the therapeutic effect of cobalamin and its derivatives against dermatitis.

However, the prior techniques that apply cobalamin and its derivatives for treating dermatitis, especially atopic dermatitis, failed in maximizing the effect partially, as follows. First, they mainly used cyanocobalamin derivatives as an active ingredient and could not maintain the effect until cyanocobalamin derivatives changed into adenosylcobalamin in the human body. Second, cobalamin is sensitive and unstable to light and heat, and the effect of the medicine could easily decrease. Third, the skin penetration rate is low with low treating effect.

To solve the aforementioned problems, there have been other attempts to prepare the external formulations such as liposome, cream or gel by using adenosylcobalamin as an active ingredient along with incorporation of skin accelerator to increase skin penetration rate of an active ingredient.

U.S. Patent No. 5,798,341 discloses a method of using cyanocobalamin, hydroxocobalamin and methylcobalamin in preparing medicine for external application, while U.S. Patent No. 6,255,294 of Allergy Limited discloses a method of delivering cobalamin and its derivatives by oral or parenteral route in forms of tablets, gum, sublingual and mucous formulations. On the other hand, U.S. Patent Application No. 10/782,827 of Audrey discloses a method of preparing tablets, injections, and preparations for skin application by using vitamin B12 in combination with copper, folic acid and vitamin C. And U.S. Patent Application No. 09/858,038 discloses a method of formulating vitamin B12 into liposome, and administering the formulation to patients with special diseases. Furthermore, Adeptsrus Holding Company (Canada) has been attempting to develop a cream for functional cosmetics containing vitamin B12 to protect skin cells and maintain water retention within skin.

However, the formulations according to the aforementioned prior arts contain cyanocobalamin, hydroxocobalamin and methylcobalamin as an active ingredient, and fail to show prompt pharmaceutical effect as the cobalamin derivatives above need to be changed into adenosylcobalamin having coenzyme function for pharmaceutical effect through the metabolism in a body. Furthermore, the attempts to formulate cobalamin and its derivatives into oral preparations, injections and transdermal preparations were not successful for the following reasons: Cobalamin or its derivatives, when orally administered, show very low bioavailability. That is, more than 90% of orally administered cobalamin or its derivatives are excreted within 48 hours with regarding to injections, there has been no specific and detailed description about the techniques to embed cyanocobalamin into liposome and formulate the injections. In particular, only small amount of cobalamin or its derivatives remains in skin when injected, which requires a long-term injections to achieve desired results. Moreover, the technique has not been developed to increase the skin penetration rate of cobalamin and its derivatives, which, in turn, would increase the therapeutical effect.

Meanwhile, International Publication No. WO2003/000010 discloses a composition comprising a fortifying amount of adenosylcobalamin for use as food, drink, supplement, etc. The composition of WO2003/000010 is provided based on the discovery that adenosylcobalamin is absorbed more efficiently than other forms of cobalamin, i.e., cyanocobalamin. However, the composition of WO2003/000010 is only for oral administration of adenosylcobalamin for use in increasing level of cobalamin in a host. WO2003/000010 did not suggest external application or external composition of adenosylcobalamin for use in treating atopic dermatitis.

U.S. Patent No. 6,274,564 discloses a composition comprising a mixture of (a) cobalamin; (b) a first isolated amino acid (c) and a second isolated amino acid. Further, U.S. Patent No. 6,274,564 discloses that the pharmaceutical composition may be administrated to an individual in an appropriate diluent or in an appropriate carrier such as liposomes. However, U.S. Patent No. 6,274,564 does not mention usefulness and importance of adenosylcobalamin as an active ingredient compared to cyanocobalamin, hydroxocobalamin or methylcobalamin. U.S. Patent No. 6,274,564 mentions that the composition is used for cobalamin deficiency. The composition of U.S. Patent No. 6,274,564 is based on cellular uptake of cobalamin increase when cobalamin is used together with the above two groups of amino acids. Regarding applications for cobalamin deficiency, U.S. Patent No. 6,274,564 describes that the composition can be used for healing of skin condition including burns, sunburn, wounds, ulcers, lacerations, herpetic eruptions, psoriasis, dermatoses and eczema. However, U.S. Patent No. 6,274,564 does not teach use of adenosylcobalamin as an active ingredient in an external composition that is beneficial for use in treating atopic dermatitis compared to cyanocobalamin, hydroxocobalamin and methylcobalamin.

EP Patent Publication No. 0256472 discloses a cosmetic composition comprising adenosylcobalamin to improve elasticity, complexion, roughness, and wrinkling of the skin. However, EP Patent Publication No. 0256472 is silent on a composition comprising adenosylcobalamin for use in the treatment of atopic dermatitis.

Thus, the present inventors completed the present invention by employing a composition comprising adenosylcobalamin as an active ingredient, and formulating the composition into a form of emulsion creams, hydrated gels and gels comprising adenosylcobalamin containing liposome particles, along with incorporation of skin accelerator, thus enabling the increase in the therapeutic effect for dermatitis.

Therefore, the present invention aims to provide a composition for external application, which comprises adenosylcobalamin as an active ingredient, thus can be used in improving the effect of skin penetration.

## DETAILED DESCRIPTION OF INVENTION

The present invention relates to a composition for external application for use in the treatment of atopic dermatitis, which comprises adenosylcobalamin as an active ingredient. The adenosylcobalamin may be loaded in liposome comprising phospholipid and cholesterol. Moreover, the composition herein may further comprise a surfactant with $C_8$-$C_{16}$ alkyl group to the aforementioned active ingredients.

Hereunder is provided a detailed description of the present invention.

The present invention relates to a composition comprising as an active ingredient adenosylcobalamin (coenzyme B12), which constitutes the coenzyme of cobalamin in pinocytosis, thereby enabling the improvement of the conventional preparation containing cobalamine and its derivative. And, this shows delayed pharmaceutical action and is very low in bioavailability as more than 90% of cobalamin or its derivative relative is excreted relative to 100% of administered dosage.

Cobalamin or its derivatives, which are currently used for treating dermatitis, have drawbacks of delayed pharmaceutical action and low treatment efficacy as they cannot show pharmaceutical action until they are transformed into coenzyme and their skin permeation rate is low, respectively. On the other hand, adenosylcobalamin, which is used as an active ingredient in the present invention, may exert a pharmaceutical action as a coenzyme without a metabolism process in the body, thus resulting in prompt pharmaceutical action. Moreover, the composition herein comprises biologically friendly skin accelerator, thus improving the skin permeation of the active ingredients and is superior for use in treating atopic dermatitis, eczema and psoriasis.

Hereunder is provided a detailed description of a method for preparing adenosylcobalamin containing liposome according to the present invention.

The Liposome particles were prepared by adding saccharides into conventionally obtained liposome, followed by freeze drying.

First of all, at least one phospholipid was selected among phosphocholine based compounds (PC), and was hydrated by mixing with at least one selected from phospholipids and cholesterol, followed by freeze drying, to provide liposome particles.

The phospholipids and cholesterol were admixed in a mixing ratio of 1-10: 1 (w/v). The aforementioned cholesterol is used to enhance the hydrophobic binding, and there may be aggregation or agglomeration between the liposome particles when the mixing ratio is outside the aforementioned range. Preferably, the concentration of the mixed phospholipid is controlled within 0.1-10 mM, and in case of being outside the aforementioned range, the embedding proportion of drugs may be lowered and the liposome particles may be aggregated or agglomerated, deteriorating the stability.

The phosphocholine based compound (PC) has 3-24 carbons in diacyl group, and preferably is at least one selected among 1,2-dipalmitoyl-sn-glycero-3-phosphocholine (DPPC), 1,2-disteroyl-sn-glycero-3-phosphocholine (DSPC), L-a-phosphatidylcholine (HSPC), 1-palmitoyl-2-glutaroyl-sn-glycero-3-phosphocholine (PGPC), 1,2-dilauroyl-sn-glycero-3-phosphocholine (DLPC), 1,2-dimyristoyl-sn-glycero-3-phosphocholine (DMPC) and 1,2-dioleyl-sn-glycero-3-phosphocholine (DOPC).

The aforementioned mixture is dissolved in a lipid-soluble solvent such as chloroform, methanol and ethanol, followed by removing the solvent with an evaporation condenser, thus providing a thin lipid layer. Ammonium sulfate solution, which may act in drug loading through the concentration difference, is added to hydrate the lipid layer, thus forming liposome. The formed liposome aforementioned is then extruded at a reduced pressure, thus providing liposome particles with a particle size of 30-400 nm, and preferably of 90-120 nm.

Adenosylcobalamin is loaded into the liposome by adding adenosylcobalamin. The non-loaded adenosylcobalamin is removed by dialysis. The loading efficiency is 70-100%.

Then, saccharide is added in thus-obtained liposome solution into the concentration of 0.01-80 mM(w/v) for 1-30 minutes followed by freeze drying, to provide liposome particles containing powdered adenosylcobalamin.

The aforementioned saccharides are monosaccharides, disaccharides or polysaccharides and not specifically limited to any type, yet some of the representative examples of the monosaccharide include mannitol, maltose, glucose, mannose and fructose, the examples of the disaccharide include maltose, sucrose and trehalose, and the examples of and the polysaccharide include sorbitol, dextrin and glucosamine. The aforementioned saccharide is dissolved in the distilled water, and the solution is prepared into the concentration of 0.01-80 mM. The concentration below 0.01 mM may deteriorate the stability of liposome in freeze-drying process and may induce aggregation or agglomeration of liposome particles, while the concentration above 80 mM would induce the increase of viscosity and cause the weakening of gel, as there is considerably larger quantity of saccharide included, in comparison to the phospholipids. The freeze drying process is divided into a freezing process at the temperature between -80 °C and -70 °C and a consecutive drying process at the temperature between -50 °C and -40 °C.

As described above, the liposome particles containing adenosylcobalamin, prepared according to the present invention, serve as the supports for maintaining the liposome forms at re-dispersion. Due to the rapid dispersion of saccharides, adenosylcobalamin may be stored for a long period of time and improved its stability, with selective control of the optimal storage temperature, specific saccharides, the concentration and the mixing ratio of liposome.

Hereunder is provided a detailed description of a method for preparing hydrogel containing adenosylcobalamin according to the present invention.

First of all, the water-soluble base is completely dissolved in the distilled water in the concentration of 3-10 wt%, and adenosylcobalamin is added into the solution to provide a uniform aqueous solution. 0.5-2wt% of emulsifying agent and 40-60 wt% of solvent are also added to the aforementioned solution, and then the distilled water is further added to balance out the amount. The aforementioned mixture is stirred with a homomixer at 3,000-6,000 rpm until it becomes uniform, thus producing gel.

Some of the examples of the water-soluble base include but are not limited to carbopol, carbomer, polyethylene glycol, polypropylene glycol, polyacrylic acid, carboxymethyl cellulose, hydroxymethyl cellulose, polyvinylpyrrolidone, gelatine, alginate salt, chitin, or chitosan derivatives and their mixture. Moreover, although it is preferred to use non-ionic surfactant as the emulsifying agent, other materials may also be used without limitation for this purpose, such as polyoxyethylene fatty acid ester, polyoxyethylene glycerine fatty acid ester, polyoxyethylene sorbitan fatty acid ester, glyceryl fatty acid ester, or their mixture. Some of the examples of the solvent include but are not limited to ethanol, isopropanol, ethyl acetate, propylene glycol, ethoxydiglycol and their mixture.

Hereunder is provided a detailed description of a method for preparing emulsion cream containing adenosylcobalamin according to the present invention.

First of all, 0.1-2.0 wt% of sodium hydroxide is completely dissolved in the distilled water of third stage, and adenosylcobalamin is also added in this aqueous solution, followed by stirring at 60-90 °C with a magnetic stirrer until adenosylcobalamin is completely dissolved, thereby producing an aqueous phase containing an active ingredient. A base forming an oil phase and an emulsifying agent are added in a concentration of 5.0-15.0 wt% and 2.0-10.0 wt% relative to the total composition, followed by stirring at 60-90 °C with a magnetic stirrer until all the ingredients are completely dissolved, thereby producing an oil phase.

The aqueous phase and the oil phase are slowly admixed at 60-90 °C and stirred with a paddle mixer for 20 minutes, followed by stirring with a homomixer at 3,000-10,000 rpm until the composition becomes uniform, while being slowly cooled down to 20-40 °C with a constant temperature bath, thereby producing emulsified cream, and the produced foam is completely removed with vacuum.

The base forming an oil phase herein includes saturated or unsaturated hydrocarbon-based oil and a mixture thereof. Some of the examples of the aforementioned saturated hydrocarbon-based oil include but not limited to liquid paraffin, paraffin wax, squalene, vaseline and other branched chain hydrocarbon-based oil, while some of the examples of the aforementioned saturated or unsaturated hydrocarbon-based oil include but not limited to natural oils, such as animal fat and vegetable oil, all of which can be used in the present invention.

In particular, another feature of the present invention is that the composition for external application herein further comprises 50-500 weight parts of a skin permeation enhancer relative to one weight part of the active ingredients, hence increasing the therapeutical effect of the active ingredients.

The contents of the aforementioned skin permeation enhancer below 50 weight parts may not be sufficient to enhance the skin absorption of the active ingredients, while the content above 500 weight parts may cause phase separation of the preparation or diminish the feeling when applied on the skin.

Among surfactants with $C_8$-$C_{16}$ alkyl group, lauryl ether based compound or polyethylene oxide (PEO) based compound is added in preparing formulation as skin permeation enhancer, in order to increase the skin-absorption and the skin-affinity. Some of the representative examples of lauryl ether based compound include, without limitation, isopropyl myristate, Brij 30, sodium lauryl sulfate, propylene glycol monolaurate, monolaurin, monostearin, monoolein, monomyristin, lauryl alcohol or polyoxyethylene-9-lauryl ether. Some of the examples of the poly ethylene oxide based compound include, without limitation, Brij 90, pluronic, sorbitan monopalmitate and sorbitan trioleate.

As described above, the present inventors ascertained that the adenosylcobalamin containing composition for external application herein has superior effect for use in treating dermatitis in comparison to the conventional composition for use in the treatment of atopic dermatiti containing cobalamin or its derivatives. This adenosylcobalamin containing composition herein is preferred to comprise 0.01-7 wt% of the active ingredients relative to 100 wt% of total composition. The content of lower than 0.01 wt% may not show satisfactory therapeutical effect, while the content of higher than 7 wt% may cause skin irritation.

Moreover, the composition herein may further comprise at least one known component such as methylcobalamin, hydroxocobalamin and cyanocobalamin besides adenosylcobalamin, thus providing equivalent or superior effect.

Furthermore, the composition herein may further comprise steroid such as dexametasone, betametasone, hydrocortisone, prednisolone and clobetasol; immunosuppressive drug such as tacrolimus, pimecrolimus and cyclosporine; and vitamin such as tretinoin, vitamin E-acetate and vitamin B5, besides the active ingredients, thus showing increased effect.

Meanwhile, the composition herein may be formulated into either medicine in the form of ointment, solution, suspension, plaster and water-containing plaster or cosmetics in the form of emulsion, lotion, cream, pack, skin lotion and soap, by using the active ingredient and pharmaceutically acceptable carrier or excipient according to the conventional methods.

Although the effective application dosage of the aforementioned active ingredients depends on the severity or the region of dermatitis as well as the age of the patient, it may be applied, for example, twice or several times a day with a dosage of 0.1-5.0 g per each application.

**Best Mode**

The present invention is described more specifically by the following Examples. Examples herein are meant only to illustrate the present invention, but in no way to limit the claimed invention.

**Example 1**

Adenosylcobalamin containing liposome herein was prepared as described below under the condition that light is blocked.

1,2-distearoyl-sn-glycero-3-phosphocholine (DPPC) and cholesterol (CHOL) were dissolved in 5 mL of chloroform into the concentration of 9.58 mg/mL and 3.19 mg/mL, respectively, followed by vacuum distillation with a rotational evaporation condenser at a temperature above the phase transition temperature (41 °C), thus providing thin lipid membrane on the round flask wall. Chloroform remaining in a flask was completely removed through vacuum drying for 24 hours.

Hydration was performed by adding 10 mL of ammonium sulfate solution (250 mM) until the lipid membrane was completely dispersed, followed by stirring for 10 minutes at an interval of 2 minutes, thus providing multi-membrane liposome. After hydration, mono-membrane liposome particles were prepared by passing through a polycarbonate membrane with porosity of 200 nm (5 times) and a polycarbonate membrane with 100 nm (5 times) by using a pressurized extruder. Ammonium sulfate containing liposome was prepared by performing membrane dialysis with ammonium sulfate that is not comprised in liposome at 4 °C for 24 hours.

After adenosylcobalamin solution was prepared by dissolving adenosylcobalamin in 10% (w/v) sucrose aqueous solution into the concentration of 1.5 mg/mL, 10 mL of the solution was added in the aforementioned ammonium sulfate containing liposome, followed by stirring at 60 °C for 2 hours, and then membrane dialysis was performed with adenosylcobalamin that is not comprised in liposome at 4 °C for 48 hours, thus producing adenosylcobalamin containing liposome.

After maltose aqueous solution (30 mM) was prepared by using the distilled water of third stage, the solution was added in the same amount of the adenosylcobalamin containing liposome solution that was produced at 4 °C, thus performing reaction, followed by freezing in a deep freezer at -77 °C for 12 hours and drying in a freeze-dryer at -45 °C for 24 hours for pulverization.

**Example 2**

Adenosylcobalamin containing gel herein was prepared as described below under the condition where light is blocked.

70.0 mg of adenosylcobalamin was exactly weighed as an active ingredient, and added into 30 mL of thedistilled water of third stage, followed by gentle agitation with magnetic stirrer to dissolve adenosylcobalamin completely. 2.2 g of carbomer 940 was completely dissolved as a base under gentle agitation while being added in a small amount. 5.0 g of polyethylene glycol (PEG) 1000, 1.35 g of diethyl amine (DEA) and 55.0 mL of ethanol were added in the solution as a base, an emulsifying agent and a solvent, respectively. Third distilled water was added to provide 100 g of total solution, followed by agitation at room temperature and 3,000 rpm with homomixer until the solution becomes uniform, thus producing adenosylcobalamin containing gel.

**Example 3**

Adenosylcobalamin containing cream herein was prepared as described below under the condition that light is blocked.

1.0 g of sodium hydroxide was completely dissolved in 70 mL of the distilled water of third stage. 70.0 mg of adenosylcobalamin was exactly weighed as an active ingredient, and added into the sodium hydroxide aqueous solution, followed by stirring with a magnetic stirrer at 90 °C until adenosylcobalamin is completely dissolved, thus producing an aqueous phase containing an active ingredient. 13 g of stearic acid (a base) and 4 g of lanolin, 2 g of sucrose fatty acid ester (an emulsifying agent) were completely dissolved with 2 g of isopropyl myristate at 90 °C with a magnetic stirrer, thus producing an oil phase.

The aqueous phase and the oil phase obtained pursuant to above were slowly admixed at 90 °C and stirred with a paddle mixer for 20 minutes, followed by stirring with a homomixer at 3,000 rpm until the solution becomes uniform, while being slowly cooled down to 40 °C with a constant temperature bath, thus producing emulsified cream, and the produced foam was completely removed with vacuum.

## Example 4

Gel containing the adenosylcobalamin containing liposome herein was prepared as described below under the condition that light is blocked.

The experiment was performed by following the same procedure as in Example 2, except by using 100 mg of powdered adenosylcobalamin containing liposome that was prepared in Example 1 as an active ingredient, instead of adenosylcobalamin.

## Comparative Example 1

Liposome was prepared by following the same procedure as in the Example 1 except the use of cyanocobalamin instead of adenosylcobalamin as an active ingredient.

## Comparative Example 2

Gel was prepared by following the same procedure as in the Example 2 except the use of cyanocobalamin instead of adenosylcobalamin as an active ingredient.

## Comparative Example 3

Cream was prepared by following the same procedure as in the Example 3 except the use of cyanocobalamin instead of adenosylcobalamin as an active ingredient.

## Comparative Example 4

Gel was prepared by following the same procedure as in the Example 4 except the use of powdered cyanocobalamin (100 mg) instead of adenosylcobalamin as an active ingredient.

## Example 5

Gel was prepared by following the same procedure as in Example 2 except the addition of Lutrol 75 (1.0 g) as skin accelerator, together with adenosylcobalamin.

## Example 6

Cream was prepared by following the same procedure as in Example 3 except the addition of Brij 30 (1.5 g) as skin accelerator together with adenosylcobalamin.

## Example 7

Gel was prepared by following the same procedure as in Example 4 except the addition of sorbitan trioleate (1.7 g) as skin accelerator together with adenosylcobalamin.

## Example 8

Gel was prepared by following the same procedure as in Example 5 except the addition of adenosylcobalamin (70 mg) and methylcobalamin (30 mg).

## Example 9

Cream was prepared by following the same procedure as in Example 6 except the addition of adenosylcobalamin (70 mg) and dexametason propionate (50 mg).

**Example 10**

Cream was prepared by following the same procedure as in Example 6 except the addition of adenosylcobalamin (70 mg) and tacrolimus hydrate (15 mg).

**Example 11**

Cream was prepared by following the same procedure as in Example 6 except the addition of adenosylcobalamin (70 mg) and tretionin (25 mg).

**Test Example 1: Evaluation of medicine for external application in anti-inflammatory activity**

Hereunder is provided a description of how to evaluate the activity for use in treating dermatitis of the adenosylcobalamin containing preparations prepared in Examples 2-4, the cyanocobalamin containing preparations prepared in Comparative Examples 2-4, preparations further containing skin accelerator preparations prepared in Examples 5-7 and preparations containing multi-components prepared in Examples 8-11.

The animal test subjects with dermatitis were prepared as follows, and the activity of suppressing edema and rubefaction was evaluated. Moreover, a base control without an active ingredient was also prepared by adding base components. Positive control medicine was prepared by 0.1 % tacrolimus ointment and 0.1 % dexametasone cream.

Preparation of animal test subject with dermatitis

7-week-old female BALB/C mice were used as test subjects. Ovalbumin and aluminium hydroxide gel was suspended in physiological saline solution into final concentration values of 2 mcg/mL and 100 mg/mL. On the first test day, 0.5 mL of each suspension was administered to the abdomen of the mice, and 0.5 mL of each suspension was further administered on the 14th day. On the 28th day, 25 mL of ovalbumin (concentration: 20 mcg/mL) was subcutaneously administered to the right external ear of the mice to induce edema. 24 hours after the edema induction, rubefaction was ascertained to take place.

Measurement of activity of suppressing edema

Base control, test preparations and positive control were applied to the external ear, i.e. edema induced area, 1 hour and 4 hours after edema induction in a dosage of 100 μL per skin area 10 cm². The thickness of external ear was measured by using a dial thickness gauge. The increase in external ear thickness was calculated by deducting the value of external ear thickness before edema induction from the value of external ear thickness after edema induction. Suppression was obtained by using the following mathematical formula and the results were provided in Table 1.

**Mathematical formula**

$$\text{Rate of suppression(\%)} = \frac{\text{Thickness increase(Control group)} - \text{Thickness increase(Experimental group)}}{\text{Thickness increase(Control group)} - \text{Thickness increase(Non-induced group)}} \times 100$$

**Table 1**

| Preparations | No. of subjects | Suppression (%) after 4 hrs | Suppression (%) after 24 hrs | Suppression (%) after 48 hrs |
|---|---|---|---|---|
| Control | 3 | 0 | 0 | 0 |
| Positive control (0.1 % tacrolimus ointment) | 5 | 45.0 | 75.2 | 100.0 |
| positive control (0.1% dexametasone cream) | 5 | 38.4 | 62.7 | 93.1 |
| Example 2 | 5 | 27.4 | 50.5 | 67.2 |
| Example 3 | 5 | 28.2 | 49.1 | 71.6 |

(continued)

| Preparations | No. of subjects | Suppression (%) after 4 hrs | Suppression (%) after 24 hrs | Suppression (%) after 48 hrs |
|---|---|---|---|---|
| Example 4 | 5 | 23.6 | 38.6 | 60.2 |
| Comp. Ex. 2 | 5 | 19.5 | 36.7 | 45.4 |
| Comp. Ex. 3 | 5 | 15.1 | 32.5 | 48.8 |
| Comp. Ex. 4 | 5 | 13.3 | 30.8 | 42.3 |
| Example 5 | 5 | 43.7 | 61.4 | 88.3 |
| Example 6 | 5 | 47.2 | 66.7 | 90.6 |
| Example 7 | 5 | 37.5 | 58.1 | 78.4 |
| Example 8 | 5 | 46.3 | 70.6 | 94.5 |
| Example 9 | 5 | 50.2 | 82.3 | 100.0 |
| Example 10 | 5 | 49.6 | 81.7 | 100.0 |
| Example 11 | 5 | 38.2 | 65.6 | 89.2 |

As set forth in Table 1, the adenosylcobalamin containing preparations of Examples 2-4 were superior to the cyanocobalamin containing preparations of Comparative Examples 2-4 in suppressing edema. Preparations further containing skin accelerator in Examples 5-7 showed more superior activity compared to the preparations of Examples 2-4. Multi-component preparations in Examples 8-11 were also superior to single-component preparations in Examples 5-7.

Evaluation of activity of suppressing edema

Base control, test preparations and positive control were applied to the external ear, i.e. edema induced area, 2 and 4 hours after edema induction in a dosage of 100 $\mu$L per skin area 10 cm$^2$. The thickness of external ear was measured with the unaided eye. The degree of rubefaction was evaluated with numerical values as follows: zero for a subject with no rubefaction, one for a subject with dark or light reddish small-sized rubefaction, three for a subject with dark reddish large-sized rubefaction, with two for a subject with rubefaction with the degree of the rebufaction value between three and one, and the results were provided in Table 1.

**Table 2**

| Preparations | No. of subjects | Degree of rubefaction after 4 hrs | Degree of rubefaction after 24 hrs |
|---|---|---|---|
| Base control | 3 | 0.9$\pm$0.1 | 0.9$\pm$0.1 |
| Positive control (0.1% tacrolimus ointment) | 5 | 0.5$\pm$0.1 | 1.1$\pm$0.2 |
| Positive control (0.1% dexametasone cream) | 5 | 1.1$\pm$0.2 | 1.2$\pm$0.1 |
| Example 2 | 5 | 1.1$\pm$0.2 | 0.9$\pm$0.1 |
| Comp. Ex. 2 | 5 | 1.1$\pm$0.2 | 0.9$\pm$0.1 |
| Example 5 | 5 | 1.1$\pm$0.2 | 1.1$\pm$0.2 |
| Example 8 | 5 | 0.9$\pm$0.1 | 1.1$\pm$0.1 |
| Example 10 | 5 | 1.1$\pm$0.2 | 0.9$\pm$0.1 |

As set forth in Table 2, the adenosylcobalamin containing preparations of Example 2 were superior to the cyanocobalamin containing preparations of Comparative Example 2 in suppressing rubefaction. Preparations further containing skin accelerator in Example 5 showed superior activity compared to the preparations of Example 2. Multi-component preparations in Example 8 and Example 10 were also more superior to single-component preparations in Example 2.

**Test Example 2: Evaluation in skin water retention**

Hereunder is provided a description of how to evaluate the skin water retention of the adenosylcobalamin containing preparations prepared in Example 2, the cyanocobalamin containing preparations prepared in Comparative Example 2, the adenosylcobalamin and methylcobalamin containing preparations prepared in Example 5 and the adenosylcobalamin and tacrolimus hydrate containing preparations prepared in Example 10. Moreover, a base control without an active ingredient was also prepared by adding base components. Positive control medicine was prepared by 0.1% tacrolimus ointment and 0.1% dexametasone cream.

Preparation of animal test subject with dermatitis

7-8 week old male nude mice were used as test subjects. 5% sodium dodecyl sulfate(SDS) was treated twice daily for 7 days to damage functions in skin a horny layer, the before value of water retention in Table 4 was measured by using water evaporation measuring device (Tewameter, Germany).

Evaluation of water retention

Test preparations, positive control preparations and base control preparations were applied twice daily for 2 days in a dosage of 100 $\mu$L per skin area 10 cm$^2$. The values of water retention were measured and provided in Table 3.

**Table 3**

| Preparations | No. of subjects | Before | After 1 day | After 2 days | After 3 days | After 4 days |
|---|---|---|---|---|---|---|
| Base control | 3 | 67±2 | 71±1 | 69±3 | 69±1 | 67±2 |
| Positive control (0.1% tacrolimus ointment) | 3 | 68±2 | 85±1 | 83±1 | 80±2 | 75±2 |
| Positive control (0.1% dexametasone cream) | 3 | 70±2 | 88±2 | 85±3 | 79±1 | 74±1 |
| Example 2 | 3 | 68±1 | 87±2 | 85±2 | 80±2 | 78±1 |
| Comp. Ex. 2 | 3 | 67±2 | 75±2 | 79±1 | 78±2 | 76±1 |
| Example 5 | 3 | 71±2 | 88±2 | 84±1 | 83±3 | 81±1 |
| Example 8 | 3 | 68±2 | 88±1 | 86±2 | 80±1 | 79±2 |
| Example 10 | 3 | 69±1 | 87±1 | 85±2 | 81±2 | 80±1 |

As set forth in Table 3, the adenosylcobalamin containing preparations of Example 2 was superior to the cyanocobalamin containing preparations of Comparative Example 2 in retaining water. Preparations further containing skin accelerator in Example 5 showed more superior activity compared to the preparations of Example 2. Multi-component preparations in Example 8 and Example 10 were also superior to single-component preparations in Example 2.

**Test Example 3: Toxicity test of adenosylcobalamin**

For repeated dose toxicity study of adenosylcobalamin (100 mg), 16-hour-fasted 4-5 week old ICR mice (5 mice each group) were selected as test subjects. 100 mg of adenosylcobalamin dissolved in 0.5% carboxymethyl cellulose (CMC) was repeatedly administered by oral route for 5 days. There was neither a dead subject nor abnormal findings such as damages to internal organs.

**Formulation Example 1: Preparation of ointment**

Hereunder is provided a description of how to prepare the ointments for external application containing adenosylcobalamin.

Contents (based on 100 g of total formulation)

(a) 0.07 g of adenosylcobalamin;
(b) 1 g of stearic acid, 10 g of monostearic acid, 4 g of monostearic acid poly(oxyethylene glycol), 1.5 g of poly(oxyethylcetostearyl ether) (20 ethylene oxide), 1.2 g of poly(oxyethylcetostearyl ether), 3 g of cetanol and 10 g of liquid paraffin; and
(c) 10 g of 1,3-butylene glycol, 6 g of glycerin and a balance of distilled water

Preparation method

The oil phase, i.e. the composition (b), was exactly weighed and placed in a supplemental tank, followed by heating up to 75 °C for dissolution. The water phase, i.e. the composition (c), was exactly weighed and placed in an emulsifying tank, followed by heating up to 75 °C for dissolution and addition of the component (a).
The oil phase was added into the emulsifying tank at vacuum condition, and stirred with a homogenizer (3500 rpm) and a pedal mixer (100 rpm), followed by cooling down to about 25 °C and aging, thus providing ointments for external application.

**Formulation Example 2: Preparation of liquids for external application**

Hereunder is provided a description of how to prepare the liquids for external application containing adenosylcobalamin.

Contents (based on 100 g of total formulation)

0.1 g of adenosylcobalamin, 0.5 g of isopropanol, 0.5 g of cetanol, 0.2 g of 1,3-butylene glycol, 0.5 g of carboxy methyl cellulose and a balance of distilled water.

Preparation method

Cetanol was exactly weighed and placed in a supplemental Tank, followed by heating up to 70 °C for dissolution. Sodium carboxy methyl cellulose, 1,3-butylene glycol and adenosylcobalamin were moisted in distilled water with stirring, and placed in a main tank, followed by heating up to 70 °C for dissolution. The solution in the supplemental tank was slowly added into the main tank and cooled down to 40 °C, followed by addition of isopropanol, and then cooled with stirring by using a pedal mixer at 50 rpm down to about 25 °C and aged, thus providing liquids for external application.

**Formulation Example 3: Preparation of suspensions for external application**

Hereunder is provided a description of how to prepare the suspensions for external application containing adenosylcobalamin and methylcobalamin.

Contents (based on 100 g of total formulation)

(a) 0.1 g of adenosylcobalamin and 0.05 g of methylcobalamin;
(b) 1.5 g of stearic acid, 1 g of cetanol, 3 g of white Vaseline, 3 g of squalene, 1.5 g of tri(caprylic acid/capronic acid) glycerin, 1.7 g of monoolefinsorbitan and 4 g of poly(ethylene glycol);
(c) 4 g of dipropylene glycol, 0.5 g of triethanol amine and 50 g of distilled water; and
(d) 8.5 g of isopropanol and balance of distilled water

Preparation method

The composition (c) was exactly weighed and placed in a main tank, followed by heating up to 70 °C for dissolution. The composition (b) was exactly weighed and placed in supplemental tank, followed by heating up to 70 °C for dissolution, and then slowly added into the main tank with a homogenizer at 2000 rpm. The composition (c) was added into a main tank, and cooled down to 40 °C with stirring by using pedal mixer at 100 rpm, followed by addition of the composition (d), and then cooled down to 25 °C with stirring by using a pedal mixer at 50 rpm and aged, thus providing suspensions for external application.

**Formulation Example 4: Preparation of plasters**

Hereunder is provided a description of how to prepare the plasters for external application containing adenosylcobalamin and tacrolimus hydrate.

Contents based on 100 g of total formulation)

    (a) 2.0 g of adenosylcobalamin and 1 g of tacrolimus hydrate;
    (b) 3 g of isopropyl myristate, 5 g of liquid paraffin, 20 g of polybudene and 25 g of 1,3-pentadiene copolymer resin;
    (c) 2 g of titanium oxide, 0.1 g of dibutylhydroxytoluene, 1 g of stearic acid polyoxyethylene sorbitan and 2 g of zinc oxide;
    (d) 7 g of caoline;
    (e) 18 g of solid natural rubber latex and 15 g of solid SBR synthesized rubber; and
    (f) 0.07 g of sodium polyacrylate, 1 g of distilled water and 0.5 g of glycerin

Preparation method

The composition (b) was exactly weighed and placed in a main tank. The temperature was elevated up to 115 °C for dissolution and maintained at 90 °C. After the addition of the composition (a), the temperature was controlled at 70 °C. The composition (c) and the composition (d) were admixed in a supplemental Tank, and added into the main tank. Moreover, the composition (f) was added in the main tank, the composition (e) was also added in the main tank at 70 °C, thus providing ointments for external application. Thus, the prepared ointments were coated over woven or non-woven fabric (100 g per 1 $m^2$), and such fabric was cut into the dimension of 10cm x 14cm, consequently providing plasters.

**Formulation Example 5: Preparation of water-retaining plasters**

Hereunder is provided a description of how to prepare the water-retaining plasters for external application containing adenosylcobalamin and dexametasone propionate.

Contents

    (a) 1.0 g of adenosylcobalamin and 0.4 g of dexametason propionate;
    (b) 25 g of D-sorbitol, 10 g of distilled water, 15 g of caoline and 1 g of titanium oxide;
    (c) 1 g of gelatin and 5 g of distilled water;
    (d) 0.2 g of sodium metaphosphate and 1 g of distilled water;
    (e) 0.2 g of magnesium hydroxyaluminate, 6 g of sodium polyacrylate, 4 g of propylene glycol, 0.5 g of acrylic acid starch(?), 1 g of castor oil, 0.5 g of monoolefinic acid polyoxyethylene sorbitan and 0.5 g of monoolefinic acid sorbitan;
    (f) 15 g of D-sorbitol(15 g) and 0.1 g of dibutylhydroxytoluene;
    (g) 3 g of methacrylic acid/acrylic acid n-butyl copolymer; and
    (h) 5 g of D-sorbitol(5 g) and 1.2 g of tartaric acid

Preparation method

The composition (b) was exactly weighed and placed in a main tank, followed by heating up to 40 °C for dissolution. Moreover, the composition (d) in the supplemental tank, which was heated and dissolved at 40 °C, was added in the main tank, and the composition (c) and (g) were also introduced, while mixing the composition in the main tank with a pedal mixer at 100 rpm. After the composition (a) and (e) were admixed and introduced, the composition (h) was slowly added to produce ointments. 12 g of ointments produced thereto were uniformly coated on non-woven fabric in a dimension of 10cm x 14cm, consequently providing plasters.

**Formulation Example 6: Preparation of skin lotions**

Hereunder is provided a description of how to prepare the skin lotions for external application containing adenosylcobalamin.

Contents (based on 100 g of total formulation)

(a) 0.2 g of adenosylcobalamin;
(b) 0.5 g of sodim carboxymethyl cellulose, 6 g of polyethylene glycol and 4 g of propylene glycol;
(c) 1 g of polyoxyethylene oleincetyl ether and 0.5 g of ojoba oil;
(d) An appropriate amount of perfume and 10 g of ethanol; and
(e) A balance of distilled water

Preparation method

The composition (e) and (b) were exactly weighed and admixed with each other, and then the mixture was added into a main tank, followed by heating up to 45 °C for dissolution. Further, while stirring the composition in the main tank with a pedal mixer at 100 rpm, the composition (a) was added, dissolved and cooled to the room temperature. The composition (d) was introduced in supplementary tank, and the composition (c) was also added, dispersed, and uniformly stirred with a pedal mixer at 300 rpm.

**Formulation Example 7: Preparation of lotions**

Hereunder is provided a description of how to prepare the lotions for external application containing adenosylcobalamin and dexametasone propionate.

Contents (based on 100 g of total formulation)

0.4 g of adenosylcobalamin, 5 g of glycerin, 10 g of isopropanol, 1 g of cetanol, 0.5 g of polyoxyethylene cetostearyl ether, 0.5 g of triethol amine, 3 g of stearic acid and a balance of distilled water

Preparation method

Cetanol, polyoxyethylene cetostearyl ether, and stearic acid were exactly weighed and added into a main tank, and then stirred with a homogenizer at 2000 rpm, followed by heating up to 70 °C for dissolution. Moreover, triethanol amine, distilled water, adenosylcobalamin and glycerin were added into the supplementary tank and heated up to 70 °C for dissolution while stirring with a pedal mixer at 50 rpm. The composition from the supplementary tank was slowly added into the main tank, and cooled down to 40 °C. The composition was abruptly cooled down to 25 °C while adding isopropyl alcohol and stirring continuously, followed by aging, consequently providing lotions.

**Industrial Applicability**

As set forth above, a composition comprising adenosylcobalamin as an active ingredient according to the present invention was ascertained to effectively suppress edema and rubefaction in the animal test subjects with dermatitis and to increase the skin penetration by comprising a skin accelerator, thus enabling the usefulness of the composition for external application for use in treating atopic dermatitis.

**Claims**

1.　Use of adenosylcobalamin for the preparation of an external composition for the treatment of atopic dermatitis, wherein the adenosylcobalamin is embedded in a liposome comprising phospholipid and cholesterol.

2.　The use of claim 1, wherein the composition comprises 0.01-7 wt% of adenosylcobalamin.

3.　The use of claim 1, wherein the composition further comprises at least one active ingredient selected from the group consisting of methylcobalamin, hydroxocobalamin and cyanocobalamin.

4. The use of claim 1 or 3, wherein the composition further comprises steroid, vitamin or immunosuppressive drug.

5. The use of claim 1, wherein weight ratio of the phospholipid and the cholesterol is 1-10 : 1.

6. The use of claim 1, wherein the phospholipid is phosphocholine having 3-24 carbons in diacyl group.

7. The use of claim 6, wherein the phospholipid is at least one selected from the group consisting of 1,2-dipalmitoyl-sn-glycero-3-phosphocholine (DPPC), 1,2-disteroyl-sn-glycero-3-phosphocholine (DSPC), L-a-phosphatidyl choline (HSPC), 1-palmitoyl-2-glutaroyl-sn-glycero-3-phosphocholine (PGPC), 1,2-dilauroyl-sn-glycero-3-phosphocholine (DLPC), 1,2-dimyristoyl-sn-glycero-3-phosphocholine(DMPC) and 1,2-dioleyl-sn-glycero-3-phosphocholine (DOPC).

8. The use of claim 1, wherein the composition comprises 50-500 weight parts of surfactant with $C_8$ -$C_{16}$ alkyl group relative to one weight part of the active ingredient.

9. The use of claim 8, wherein the surfactant is lauryl ether based compound or poly(ethylene oxide) based compound.

10. The use of claim 9, wherein the lauryl ether based compound is at least one compound selected from the group consisting of isopropyl myristate, Brij 30, sodium lauryl sulfate, propylene glycol monolaurate, monolaurin, monostearin, monoolein, monomyristin, lauryl alcohol and polyoxyethylene 9 lauryl ether.

11. The composition of claim 9, wherein the poly (ethylene oxide) based compound is at least one compound selected from the group consisting of Brij 90, pluronic, sorbitan monopalmitate and sorbitan trioleate.

12. The composition of claim 1, wherein the composition is formulated into one preparation selected from the group consisting of hydrogel, emulsion cream, liposome containing hydrogel, ointment, solution, suspension, plaster, water containing plaster, skin lotion or lotion.

**Patentansprüche**

1. Verwendung von Adenosylcobalamin für die Herstellung einer äußerlichen Zusammensetzung für die Behandlung von atopischer Dermatitis, wobei das Adenosylcobalamin in ein Liposom eingelagert ist, das Phospholipid und Cholesterol enthält.

2. Verwendung nach Anspruch 1, wobei die Zusammensetzung 0,01-7 Gew.-% Adenosylcobalamin enthält.

3. Verwendung nach Anspruch 1, wobei die Zusammensetzung weiterhin mindestens einen aktiven Inhaltsstoff, ausgewählt aus der Gruppe bestehend aus Methylcobalamin, Hydroxocobalamin und Cyanocobalamin, enthält.

4. Verwendung nach Anspruch 1 oder 3, wobei die Zusammensetzung weiterhin Steroid, Vitamin oder immunsuppressiven Wirkstoff enthält.

5. Verwendung nach Anspruch 1, wobei das Gewichtsverhältnis von Phospholipid zu Cholesterol 1-10:1 ist.

6. Verwendung nach Anspruch 1, wobei das Phospholipid ein Phosphocholin mit 3-24 Kohlenstoffatomen in der Diacylgruppe ist.

7. Verwendung nach Anspruch 6, wobei das Phospholipid mindestens eins, ausgewählt aus der Gruppe bestehend aus 1,2-Dipalmitoyl-sn-glycero-3-phosphocholin (DPPC), 1,2-Disteroyl-sn-glycerophosphocholin (DSPC), L-a-Phosphatidylcholin (HSPC), 1-Palmitoyl-2-glutaroyl-sn-glycero-3-phosphocholin (PGPC), 1,2-Dilauroyl-sn-glycero-3-phosphocholin (DLPC), 1,2-Dimyristoyl-sn-glycero-3-phosphocholin (DMPC) und 1,2-Dioleyl-sn-glycero-3-phosphocholin (DOPC), ist.

8. Verwendung nach Anspruch 1, wobei die Zusammensetzung 50-500 Gewichtsteile Tensid mit $C_8$-$C_{16}$ Alkylgruppen bezogen auf ein Gewichtsteil des aktiven Inhaltsstoffes enthält.

**9.** Verwendung nach Anspruch 8, wobei das Tensid eine Verbindung auf Laurylether- oder Poly(ethylenoxid)-Basis ist.

**10.** Verwendung nach Anspruch 9, wobei die Verbindung auf Laurylether-Basis mindestens eine Verbindung ausgewählt aus der Gruppe bestehend aus Isopropylmyristat, Brij 30, Natriumlaurylsulfat, Propylenglykolmonolaureat, Monolaurin, Monostearin, Monoolein, Monomyristin, Laurylalkohol und Polyoxyethylen-9-laurylether, ist.

**11.** Zusammensetzung nach Anspruch 9, wobei die Verbindung auf Poly(ethylenoxid)-Basis mindestens eine Verbindung, ausgewählt aus der Gruppe bestehend aus Brij 90, Pluronic, Sorbitanmonopalmitat und Sorbitantrioleat, ist.

**12.** Zusammensetzung nach Anspruch 1, wobei die Zusammensetzung in eine Zubereitung, ausgewählt aus einer Gruppe bestehend aus Hydrogel, Emulsionscreme, Liposom enthaltendes Hydrogel, Balsam, Lösung, Suspension, Pflaster, Wasser enthaltendes Pflaster, Hautlotion oder Lotion, formuliert ist.

**Revendications**

**1.** Utilisation d'adénosyl-cobalamine pour la préparation d'une composition externe destinée au traitement de la dermatite atopique, ladite adénosyl-cobalamine étant incorporée dans un liposome comprenant du phospholipide et du cholestérol.

**2.** Utilisation selon la revendication 1, où la composition comprend 0,01-7% en poids d'adénosyl-cobalamine.

**3.** Utilisation selon la revendication 1, où la composition comprend en outre au moins un principe actif choisi dans le groupe constitué par la méthylcobalamine, l'hydroxocobalamine et la cyanocobalamine.

**4.** Utilisation selon la revendication 1 ou 3, où la composition comprend en outre un stéroïde, une vitamine ou un médicament immunosuppresseur.

**5.** Utilisation selon la revendication 1, où le rapport pondéral du phospholipide au cholestérol est de 1-10 : 1.

**6.** Utilisation selon la revendication 1, où le phospholipide est une phosphocholine ayant 3-24 atomes de carbone dans le groupe diacyle.

**7.** Utilisation selon la revendication 6, où le phospholipide est au moins un phospholipide choisi dans le groupe constitué par la 1,2-dipalmitoyl-sn-glycéro-3-phosphocholine (DPPC), la 1,2-distéroyl-sn-glycéro-3-phosphocholine (DSPC), la L-a-phosphatidylcholine (HSPC), la 1-palmitoyl-2-glutaroyl-sn-glycéro-3-phosphocholine (PGPC), la 1,2-dilauroyl-sn-glycéro-3-phosphocholine (DLPC), la 1,2-dimyristoyl-sn-glycéro-3-phosphocholine (DMPC) et la 1,2-dioléyl-sn-glycéro-3-phosphocholine (DOPC).

**8.** Utilisation selon la revendication 1, où la composition comprend 50-500 parties en poids d'agent tensioactif ayant un groupe alkyle en $C_8$-$C_{16}$ par rapport à une partie en poids du principe actif.

**9.** Utilisation selon la revendication 8, où l'agent tensioactif est un composé à base d'éther laurylique ou un composé à base de poly(oxyde d'éthylène).

**10.** Utilisation selon la revendication 9, où le composé à base d'éther laurylique est au moins un composé choisi dans le groupe constitué par le myristate d'isopropyle, Brij 30, le laurylsulfate de sodium, le monolaurate de propylèneglycol, la monolaurine, la monostéarine, la monooléine, la monomyristine, l'alcool laurylique et l'éther laurylique de polyoxyéthylène(9).

**11.** Composition selon la revendication 9, dans laquelle le composé à base de poly(oxyde d'éthylène) est au moins un composé choisi dans le groupe constitué par Brij 90, Pluronic, le monopalmitate de sorbitane et le trioléate de sorbitane.

**12.** Composition selon la revendication 1, ladite composition étant formulée en une préparation choisie dans le groupe constitué par un hydrogel, une crème-émulsion, un hydrogel contenant des liposomes, une pommade, une solution, une suspension, un emplâtre, un emplâtre aqueux, une lotion cutanée ou une lotion.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5798341 A
- US 6255294 B
- US 782827 A
- US 858038 A
- WO 2003000010 A
- US 6274564 B
- EP 0256472 A

**Non-patent literature cited in the description**

- **YAMASHIKI M. ; NISHIMURA A. ; KOSAKA Y.** *J. Clin. Lab. Immunol.,* 1992, vol. 37, 173-182
- **STACKER M. ; PIECK C. ; STOERB C. ; NIEDNER R. ; HARTUNG J. ; ALTMEYER P.** *Br. J. Dermatol.,* 2004, vol. 150, 977-983